# EUROPEAN PATENT APPLICATION

(11) **EP 4 205 678 A1**
(43) Date of publication of application: **05.07.2023**
(21) Application number: 22216857.7
(22) Date of filing: 28.12.2022
(51) Int. Cl.: A61B 18/14

(54) **CATHETER BALLOON HAVING INCREASED RESILIENCE TO INTERNAL PRESSURIZATION**

(30) Priority: 29.12.2021 US 202163294823 P; 01.12.2022 US 202218072905
(71) Applicant: Biosense Webster (Israel) Ltd., Yokneam, 2066717 (IL)
(72) Inventor: VU, Audrey, Irvine, 92618 (US); RODRIGUEZ, Jasson, Irvine, 92618 (US)
(74) Representative: Carpmaels & Ransford LLP

(57) **Abstract**

A catheter balloon comprises an ellipsoidal membrane. A plurality of flexible circuit strips, each of which comprises a substrate and a contact electrode, are disposed about the membrane. A coating is disposed atop at least the outer surface of the membrane and may also be disposed atop a portion of each of the substrates. The coating may comprise a dielectric material, such as parylene. The coating may increase the smoothness of the balloon. When subject to internal pressures that expand the balloon, the coating may also increase the resilience of the balloon relative to a balloon that lacks the coating as determined by changes in the balloon's diameter before and after expansion.

## Description

### CROSS-REFERENCE TO CO-PENDING APPLICATION

The present application claims priority under 35 U.S.C. § 119 to U.S. Patent Application No. 63/294,823, filed December 29, 2021. The entire content of this application is incorporated by reference herein in its entirety.

### FIELD

The subject matter disclosed herein relates to electrophysiologic catheters, particularly those capable of ablating cardiac tissue via electrodes disposed on a balloon surface.

### BACKGROUND

Ablation of cardiac tissue has been used to treat cardiac arrhythmias. Ablative energies are typically provided to cardiac tissue by a tip portion, which can deliver ablative energy alongside the tissue to be ablated. Some of these catheters administer ablative energy from various electrodes disposed on or incorporated into three-dimensional structures, e.g., wire baskets and balloons.

### SUMMARY OF THE DISCLOSURE

A catheter balloon comprises an ellipsoidal membrane including a proximal end and a distal end, and an outer surface. A plurality of flexible circuit strips (*e.g.,* ten) are disposed about the membrane. Each of the flexible circuit strips extend from the proximal end of the membrane toward the distal end of the membrane. Additionally, each comprises a substrate and a contact electrode. The substrate includes an outer surface and an inner surface disposed atop the outer surface of the membrane. The contact electrode is disposed on the outer surface of the substrate so that the contact electrode is exposable to an environment, e.g., a cardiac environment. Each of the contact electrodes has fishbone configuration including a plurality of protrusions extending away from a main body.

A coating is disposed atop at least the outer surface of the membrane. The coating may also be disposed atop a portion of each of the substrates. For example, the coating may be disposed on the substrate between a first protrusion and a second protrusion of the plurality of protrusions.

The coating may comprise a dielectric material, such as parylene. A thickness of the coating may be between about one micron and about five microns, *e.g.,* about three microns. Further, the coating may increase the smoothness of the balloon. For example, a difference between a first distance from the outer surface of the membrane to an outer surface of the coating and a second distance from the outer surface of the membrane to the outer surface of the contact electrode may be less than about 5 micrometers, *e.g.,* less than about 1 micrometer or less than about 0.5 micrometers.

The coating increases the resilience of the balloon relative to a balloon that lacks the coating. For example, the membrane may have a first diameter (*e.g.,* about thirty millimeters) when subject to a first internal pressure of less than about one pound per square inch, and in which the membrane has a second diameter that is greater than the first diameter when subject to a second internal pressure of greater than about one pound per square inch. When the balloon is coated, the second diameter is less than 0.5% greater than the first diameter when the second internal pressure is between about 1 pound per square inch and about 2.5 pounds per square inch, the second diameter is less than 1.25% greater than the first diameter when the second internal pressure is between about 2.5 pounds per square inch and about 4 pounds per square inch, and the second diameter is less than 2% greater than the first diameter when the second internal pressure is between about 4 pounds per square inch and about 5.5 pounds per square inch.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 depicts a schematic illustration of an invasive medical procedure;
Figure 2 depicts a top view of a catheter with a balloon in an expanded state, in use with a lasso catheter;
Figure 3 depicts a perspective view of a distal end of the catheter of Figure 2, reflecting the balloon as including a reinforcement comprising a reinforcement component;
Figure 4 depicts a perspective view of a distal end of the catheter of Figure 2, reflecting structure of the catheter internal to the balloon;
Figure 5 depicts a perspective detail view of a strip of a flex circuit electrode assembly on the balloon of Figure 3;
Figure 6 depicts a top view of the strip reflected in Figure 5;
Figure 6A depicts a detailed view of a portion of the strip as depicted in Figure 6;
Figure 6B depicts a cross-section view of a portion of the detailed view depicted in Figure 6A, taken along the section line 6B-6B;
Figure 7A depicts an alternate detailed view of a portion of the strip as depicted in Figure 6;
Figure 7B depicts a cross-section view of a portion of the detailed view depicted in Figure 7A, taken along the section line 7B-7B;
Figure 8A reflects a photograph of a first specimen of the balloon in an expanded configuration; and
Figure 8B reflects a photograph of a second specimen of the balloon in an expanded configuration.

### MODES OF CARRYING OUT THE INVENTION

The following detailed description should be read with reference to the drawings, in which like elements in different drawings are similarly numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

### Overview

Ablation of cardiac tissue to correct a malfunctioning heart is a well-known procedure. Typically, to successfully ablate, cardiac electropotentials need to be measured at various locations of the myocardium. In addition, temperature measurements during ablation provide data enabling the efficacy of the ablation to be measured. Typically, for an ablation procedure, the electropotentials and the temperatures are measured before, during, and after the actual ablation.

An ablation catheter may include a lumen, and a balloon may be deployed through the catheter lumen. A multi-layer flexible metal structure is attached to an exterior wall or membrane of the balloon. The structure comprises a plurality of electrode groups arranged circumferentially about the longitudinal axis, where each electrode group comprises multiple ablation electrodes, typically arranged longitudinally.

Each electrode group may also include at least one micro-electrode that is insulated physically and electrically from the ablation electrodes in its group. Each electrode group may also include at least a thermocouple. In some embodiments, each electrode group includes a micro-electrode and a thermocouple formed at a common location. Using a single catheter, with the three functionalities of ability to perform ablation, electropotential measurement, and temperature measurement, simplifies cardiac ablation procedures.

### System Description

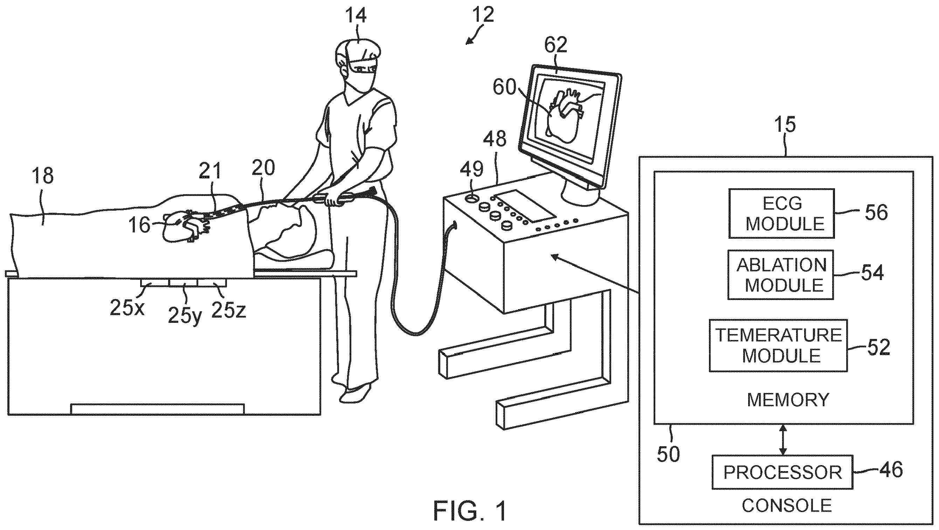

Figure 1 is a schematic illustration of an invasive medical procedure using apparatus 12, according to an embodiment. The procedure is performed by a medical professional 14, and, by way of example, the procedure in the description hereinbelow is assumed to comprise ablation of a portion of a myocardium 16 of the heart of a human patient 18. However, it is understood that embodiments disclosed herein are not merely applicable to this specific procedure, and may include substantially any procedure on biological tissue or on non-biological materials.

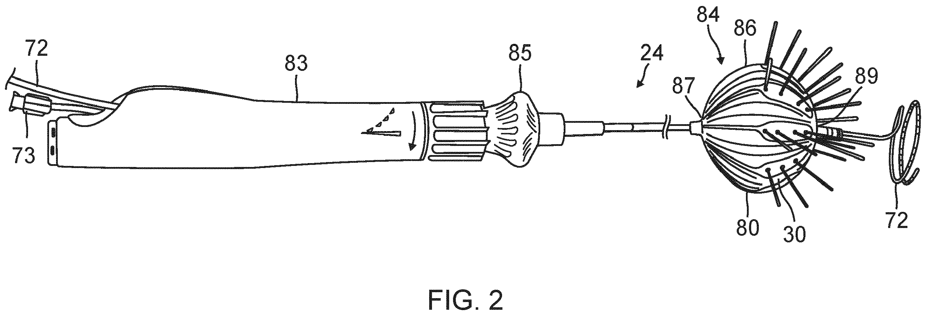

To perform the ablation, medical professional 14 inserts a probe 20 into a sheath 21 that has been pre-positioned in a lumen of the patient. Sheath 21 is positioned so that a distal end 22 of probe 20 enters the heart of the patient. A diagnostic/therapeutic catheter 24 (e.g., a balloon catheter), reflected in Figure 2, is at least partially disposed in a lumen 23 of probe 20, such that it can be deployed through lumen 23 to exit from a distal end of the probe 20.

As shown in Figure 1, apparatus 12 is controlled by a system processor 46, which is in an operating console 15 of the apparatus. Console 15 comprises controls 49 which are used by professional 14 to communicate with the processor. During the procedure, the processor 46 typically tracks a location and an orientation of the distal end 22 of the probe 20, using any method known in the art. For example, processor 46 may use a magnetic tracking method, wherein magnetic transmitters 25X, 25Y and 25Z external to the patient 18 generate signals in coils positioned in the distal end of the probe 20. The CARTO^{®} system (available from Biosense Webster, Inc. of Irvine, California) uses such a tracking method.

The software for the processor 46 may be downloaded to the processor in electronic form, over a network, for example. Alternatively, or additionally, the software may be provided on non-transitory tangible media, such as optical, magnetic, or electronic storage media. The tracking of the distal end 22 is may be displayed on a three-dimensional representation 60 of the heart of the patient 18 on a screen 62. However, it may be displayed two-dimensionally, e.g., by fluoroscopy or MRI.

To operate apparatus 12, the processor 46 communicates with a memory 50, which has many modules used by the processor to operate the apparatus. Thus, the memory 50 comprises a temperature module 52, an ablation module 54, and an electrocardiograph (ECG) module 56, the functions of which are described below. The memory 50 typically comprises other modules, such as a force module for measuring the force on the distal end 22, a tracking module for operating the tracking method used by the processor 46, and an irrigation module allowing the processor to control irrigation provided into balloon 80 via fluid connection 73. For simplicity, such other modules are not illustrated in Figure 1. The modules may comprise hardware as well as software elements. For example, module 54 may include a radio-frequency generator with at least one output or output channel, e.g., ten outputs or ten output channels. Each of the outputs may be separately and selectively activated or deactivated by a switch. That is, each switch may be disposed between the signal generator and a respective output. Thus, a generator with ten outputs would include ten switches. These outputs may each be individually coupled to electrodes on an ablation catheter, e.g., the ten electrodes 33 on balloon 80, described in further detail below. Such an electrical connection may be achieved by establishing an electrical path between each output and each electrode. For example, each output may be connected to a corresponding electrode by one or more wires or suitable electrical connectors. Thus, in some embodiments, an electrical path may include at least one wire. In some embodiments, the electrical path may further include an electrical connector and at least a second wire. Thus, electrodes 33 may be selectively activated and deactivated with the switches to receive radiofrequency energy separately from each of the other electrodes.

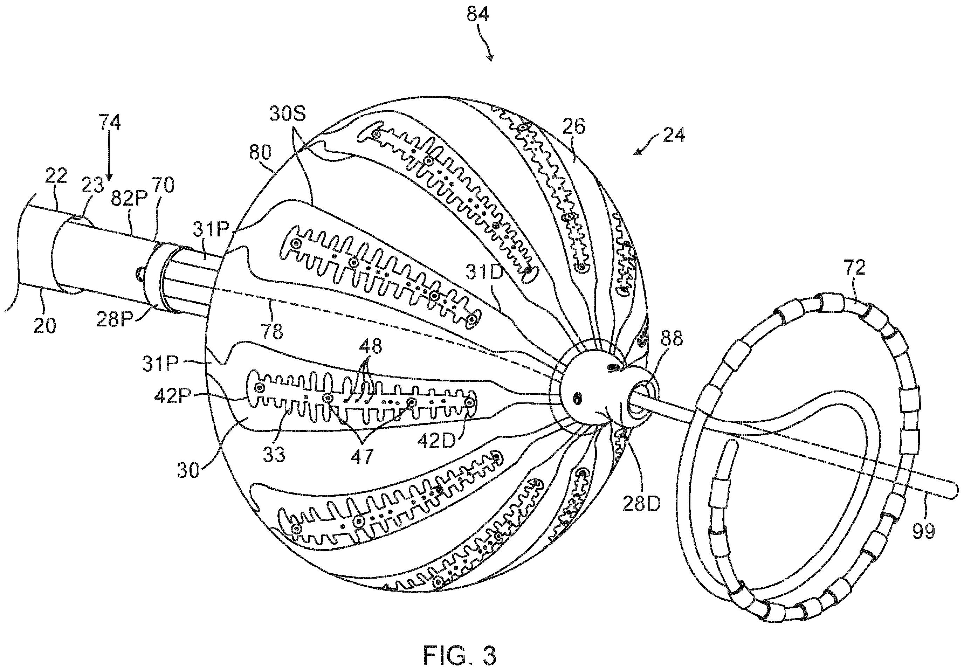

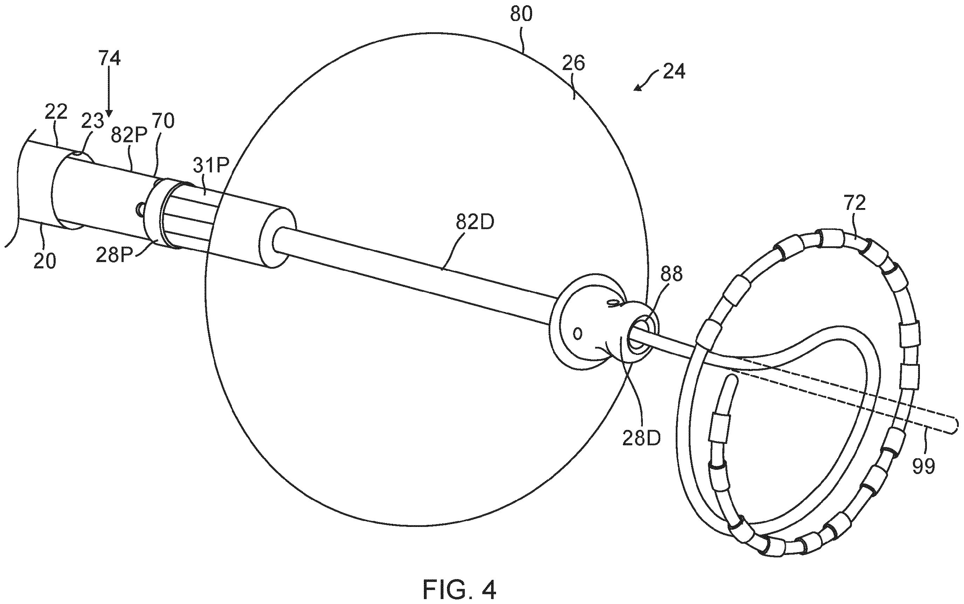

Figure 3 is a schematic perspective view of the diagnostic/therapeutic catheter 24 showing balloon 80 in an expanded configuration. The diagnostic/therapeutic catheter 24 is supported by a tubular shaft 70 having a proximal or first shaft portion 82P, a distal or second shaft portion 82D, and a distal shaft end 88. As depicted in Figure 4, which is similar to Figure 3, but with external structures on balloon 80 hidden, distal or second shaft portion 82D is disposed at least partially within the proximal or first shaft portion 82P in a telescoping relationship therewith. The shaft 70 also includes a hollow central tube 74, which permits a catheter to pass therethrough and past the distal shaft end 88. The catheter may be a focal linear catheter or a lasso catheter 72, as illustrated. The lasso catheter 72 may be inserted into the pulmonary vein to position the diagnostic/therapeutic catheter 24 correctly with respect to an ostium prior to ablation of the ostium. The distal lasso portion of the catheter 72 is typically formed of shape-memory retentive material such as nitinol. It is understood that the diagnostic/therapeutic catheter 24 may also be used with a linear or focal catheter 99 (as shown in broken lines in Figure 3) in the PV or elsewhere in the heart. The focal catheter 99 may include a force sensor at its distal tip. Suitable force sensing distal tips are disclosed in U.S. Patent Nos. 8,357,152 and 10,688,278, the entire contents of which are incorporated by reference herein. Any catheter used in conjunction with the diagnostic/therapeutic catheter may have features and functions, including, for example, pressure sensing, ablation, diagnostic, e.g., navigation and pacing.

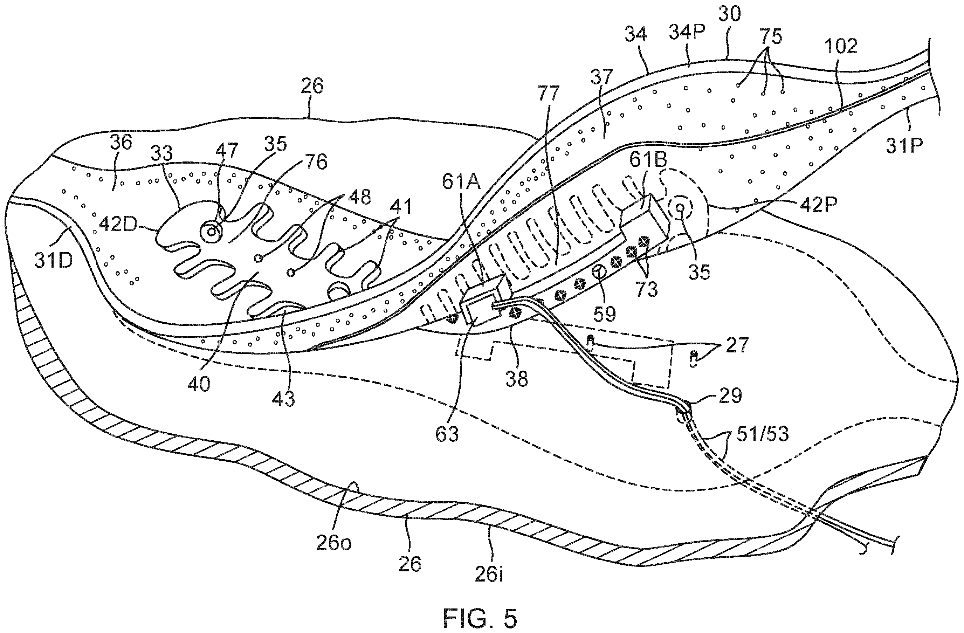

With further reference to Figure 5, balloon 80 of the diagnostic/therapeutic catheter 24 comprises a membrane 26 of a bio-compatible material, for example, formed from a plastic such as polyethylene terephthalate (PET), polyurethane, Pellethane^{®} or PEBAX^{®}. Membrane 26 has an outer surface 26o and an inner surface 26i. Membrane 26, and thus balloon 80, have a proximal end 87 and a distal end 89 at distal shaft end 88.

The shaft 70 and the distal shaft end 88 define a longitudinal axis 78 of the balloon 80. The balloon 80 is deployed, in a collapsed configuration, via the lumen 23 of the probe 20. A proximal end of membrane 26 of balloon 80 is attached to first or proximal shaft portion 82P and a distal end of membrane 26 of balloon 80 is attached to second or distal shaft portion 82D, proximate to distal shaft end 88. Balloon 80 may be expanded to an expanded configuration after exiting from the distal end 22 by moving distal shaft end 88 proximally to shorten the distance between the distal end 89 of balloon 80 and proximal end 87 of balloon 80, and thus increase the width of balloon 80, i.e., by telescoping distal shaft portion 82D proximally in the proximal shaft portion 82P. Passing irrigation fluid into balloon 80 via fluid connection 73 may further expand balloon 80. Balloon 80 may be returned to its collapsed configuration by ceasing the irrigation and then moving distal shaft end 88 away from proximal end 87 to decrease the width of and extend the length of balloon 80, i.e., by telescoping distal shaft portion 82D distally in proximal shaft portion 82P. This telescopic motion between the first shaft portion and the second shaft portion may be controlled by knob 85 of control handle 83, shown in Figure 2. Specifically, knob 85 may be rotated in a first direction to extend distal shaft portion 82D distally, and thus move distal shaft end 88 distally, whereas knob 85 may be rotated in the opposite direction to withdraw distal shaft portion 82D proximally, and thus move distal shaft end 88 proximally. Knob 85 may further include locking features, such as detents, to maintain distal shaft end at its most proximal location and at its most distal location. Such locking features help prevent balloon 80 from collapsing somewhat out of its expanded configuration during ablation and from expanding somewhat out if its collapsed configuration during withdrawal into lumen 23 of probe 20. Further description concerning transitioning the balloon between a collapsed configuration and an expanded configuration is set forth in U.S. Patent Application No. 15/827,111, published as U.S. Patent Application Publication No. 2018/0161093. The entire content of this application is incorporated by reference herein in its entirety.

The membrane 26 supports and carries a combined electrode and temperature sensing member which is constructed as a multi-layer flexible circuit electrode assembly 84. The "flex circuit electrode assembly" 84 may have many different geometric configurations. In the illustrated embodiment, the flex circuit electrode assembly 84 has a plurality of radiating flexible-circuit strips 30. The strips 30 are evenly distributed about outer membrane surface 26o of balloon 80. Each comprises a substrate 34 that has wider proximal portion that gradually tapers to a narrower distal portion. As seen in Figure 3, flexible circuit electrode assembly 84 may include ten strips 30.

Each substrate 34 has a proximal tail 31P proximal to the wider proximal portion and a distal tail 31D distal of the narrower distal portion. As described below, distal tail portion 31D may extend up to distal shaft end 88 to be secured thereunder. Substrate 34 may be bonded to membrane 26 with an adhesive, such as an epoxy. Some adhesive may be disposed between inner surface 37 of substrate 34 and membrane 26.

The flex circuit electrode assembly 84 is described with respect to one of its strips 30 as shown in Figure 5. The flex circuit electrode assembly 84 includes a flexible and resilient sheet substrate 34, constructed of a non-electrically conductive bio-compatible material, for example, polyimide. In some embodiments, the sheet substrate 34 has a greater heat resistance (or a higher melting temperature) compared to that of the balloon membrane 26. In some embodiments, the substrate 34 is constructed of a thermoset material having a decomposition temperature that is higher than the melting temperature of the balloon membrane 26 by approximately 100 degrees Celsius or more. The substrate may have a thickness of between about 15 micrometers to about 25 micrometers, e.g., about 20 micrometers.

The substrate 34 is formed with one or more irrigation pores or apertures 35 that are in alignment with the irrigation apertures 27 of the balloon member 26 so that fluid passing through the irrigation apertures 27 and 35 can pass to the ablation site on the ostium. Substrate 34 may be cut to shape by, and the irrigation pores 35 formed by, any suitable manufacturing technique, such as laser cutting.

The substrate 34 has a first or outer surface 36 facing away from the balloon membrane 26, and a second or inner surface 37 facing the balloon membrane 26. On its outer surface 36, the substrate 34 supports and carries the contact electrodes 33 that may be exposable to an environment, such as an interior of a portion of a heart, and particularly adapted for tissue contact with a pulmonary vein ostium. On its inner surface 37, the substrate 34 supports and carries a wiring electrode 38. The contact electrode 33 delivers RF energy to the ostium during ablation or is connected to a thermocouple junction for temperature sensing of the ostium. In the illustrated embodiment, the contact electrode 33 has a longitudinally elongated portion 40 and a plurality of thin transversal linear portions or protrusions 41 extending generally perpendicularly from each lateral side of the elongated portion 40 between enlarged proximal and distal ends 42P and 42D, generally evenly spaced therebetween. The elongated portion 40 has a greater width and each of the protrusions has a generally uniform lesser width. Accordingly, the configuration or trace of the contact electrode 33 may resemble a "fishbone" with protrusions 41 being "fish bones" of varying lengths extending from a main body of the electrode, but it should be noted that the invention is not limited to such configuration. In contrast to an area or "patch" ablation electrode, the protrusions 41 of the contact electrode 33 advantageously increase the circumferential or equatorial contact surface of the contact electrode 33 with the ostium while void regions 43 between adjacent protrusions 41 advantageously allow the balloon 80 to collapse inwardly or expand radially as needed at locations along its equator. In the illustrated embodiment, the protrusions 41 have different lengths, some being longer, others being shorter. For example, the plurality of protrusions includes a distal protrusion, a proximal protrusion and protrusions therebetween, where each of the protrusions in between has a shorter adjacent protrusion. For example, each protrusion has a length different from its distal or proximal immediately adjacent neighboring protrusion(s) such that the length of each protrusion generally follows the tapered configuration of each substrate 34. In the illustrated embodiment, there are 22 protrusions extending across (past each lateral side of) the elongated portion 40, with the longest protrusion being the third protrusion from the enlarged proximal end 42P. In some embodiments, contact electrode 33 includes an outer or contact layer 76 and an inner or seed layer 77 between contact layer and substrate 34. Contact layer 76 may comprise gold. Seed layer 77 may comprise titanium, tungsten, palladium, silver, or combinations thereof. Seed layer 77 may be sputtered onto substrate 34 and contact layer 76 may be sputtered, plated, or a combination thereof, onto the seed layer. The thickness of seed layer 77 may be between about 250 angstroms to about 350 angstroms, e.g., about 300 angstroms. The thickness of contact layer 76 may be between about .75 micrometers to about 1.25 micrometers, e.g., about 1 micrometer. Whatever material seed layer 77 comprises, e.g., titanium, it should be readily bondable to the material of substrate 34, e.g., polyimide.

Formed within the contact electrode 33 are one or more exclusion zones 47, each surrounding an irrigation aperture 35 formed in the substrate 34. The exclusion zones 47 are voids purposefully formed in the contact electrode 33 so as to avoid damage to the contact electrode 33 during construction of the electrode assembly 84 in accommodating the irrigation apertures 35 at their locations and in their function.

Also formed in the contact electrode 33 are one or more conductive blind vias 48 which are conductive or metallic formations that extend through through-holes in the substrate 34 and are configured as electrical conduits connecting the contact electrode 33 on the outer surface 36 and the wiring electrode 38 on the inner surface 37. It is understood that "conductive" is used herein interchangeably with "metallic" in all relevant instances. For example, the via may be fabricated by plating gold through the through-holes in substrate 34. Preferably, the thickness blind via 48 is less than or approximates the thickness of substrate 34. For example, the thickness of blind via 48 may be between about 4 micrometers and about 25 micrometers, e.g., about 10 micrometers to about 14 micrometers.

In the illustrated embodiment, the contact electrode 33 measures longitudinally between about 0.1 inch and 1.0 inch, and preferably between about 0.5 inch and 0.7 inch, and more preferably about 0.57 inch, and has four exclusion zones 47 and nine blind vias 48.

On the inner surface 37 of the substrate 34, the wiring electrode 38 is generally configured as an elongated body generally similar in shape and size to the elongated portion 40 of the contact electrode 33. The wiring electrode 38 loosely resembles a "spine" and also functions as a spine in terms of providing a predetermined degree of longitudinal rigidity to each substrate 34 of the electrode assembly 84. The wiring electrode 38 is positioned such that each of the blind vias 48 is in conductive contact with both the contact electrode 33 and the wiring electrode 38. In the illustrated embodiment, the two electrodes 33 and 38 are in longitudinal alignment with other, with all nine blind vias 48 in conductive contact with both electrodes 33 and 38. In some embodiments, the wiring electrode 38 has an inner portion of copper and a peripheral portion of gold.

The wiring electrode 38 is also formed with its exclusion zones 59 around the irrigation apertures 35 in the substrate 34. The wiring electrode 38 is further formed with solder pad portions, at least one active 61A, and there may be one or more inactive solder pad portions 61B. The solder pad portions 61A and 61B are extensions from a lateral side of the elongated body of the wiring electrode 38. In the illustrated embodiment, an active solder pad portion 61A is formed at about a mid-location along the elongated body, and a respective inactive solder pad portion 61B is provided at each of the enlarged distal end 42D and the enlarged proximal end 42P.

Attached, e.g., by a solder weld 63, to the active solder pad portion 61A are the wire pair, e.g., a constantan wire 51 and a copper wire 53. The copper wire 53 provides a lead wire to the wiring electrode 33, and the copper wire 53 and the constantan wire 51 provide a thermocouple whose junction is at solder weld 63. The wire pair 51/53 are passed through a through-hole 29 formed in the membrane 26. It is understood that, in other embodiments in the absence of the through-hole 29, the wire pair 51/53 may run between the membrane 26 and the substrate 34 and further proximally between the membrane 26 and the proximal tail 31P until the wire pair 51/53 enters the tubular shaft 70 via another through-hole (not shown) formed in the tubular shaft sidewall closer to the proximal ring 28P.

The substrates 34 are affixed to the balloon membrane 26 such that the outer surface 36 of the substrate 34 is exposed and the inner surface 37 of the substrate 34 is affixed to the balloon membrane 26, with the wiring electrode 38 and wire pair 51/53 sandwiched between the substrate 34 and the balloon membrane 26. The irrigation apertures 35 in the substrate 34 are aligned with the irrigation apertures 27 in the balloon membrane 26. The exclusion zones 59 in the wiring electrode 38 and the exclusion zones 47 in the contact electrode 33 are concentrically aligned with each other, as well as with the irrigation apertures 27 and 35 in balloon 26 and substrate 34, respectively.

Further details on constructing a diagnostic/therapeutic catheter in accordance with the foregoing disclosure may be found in U.S. Patent Application No. 15/360,966, published as U.S. Patent Application Publication No. 2017/0312022. The entire content of this application is incorporated by reference herein in its entirety.

### Increased Resilience

Through ongoing research and product development efforts concerning the subject matter described above, Applicant has determined that the overall surface area of contact electrodes 33 that contacts tissue, e.g., tissue of a pulmonary vein ostium, may be maximized by increasing the resilience of balloon 80. As used herein, the phrase "increasing the resilience" means reducing the flexibility of balloon 80 such that the material of balloon 80 stretches little if at all in response to expansion pressures generated by the irrigation fluid that expands balloon 80 following telescopic movement of distal shaft portion 82D proximally in proximal shaft portion 82P, described above.

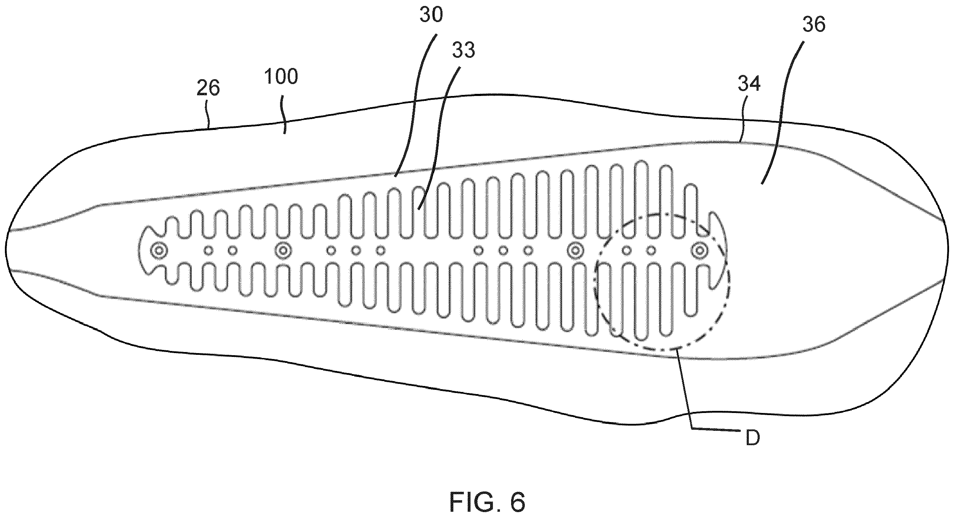

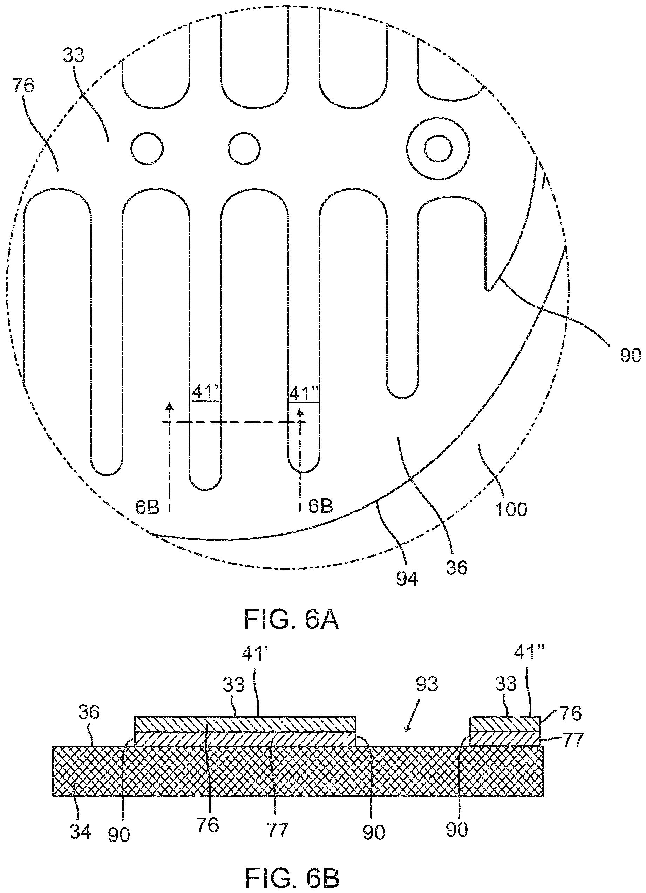

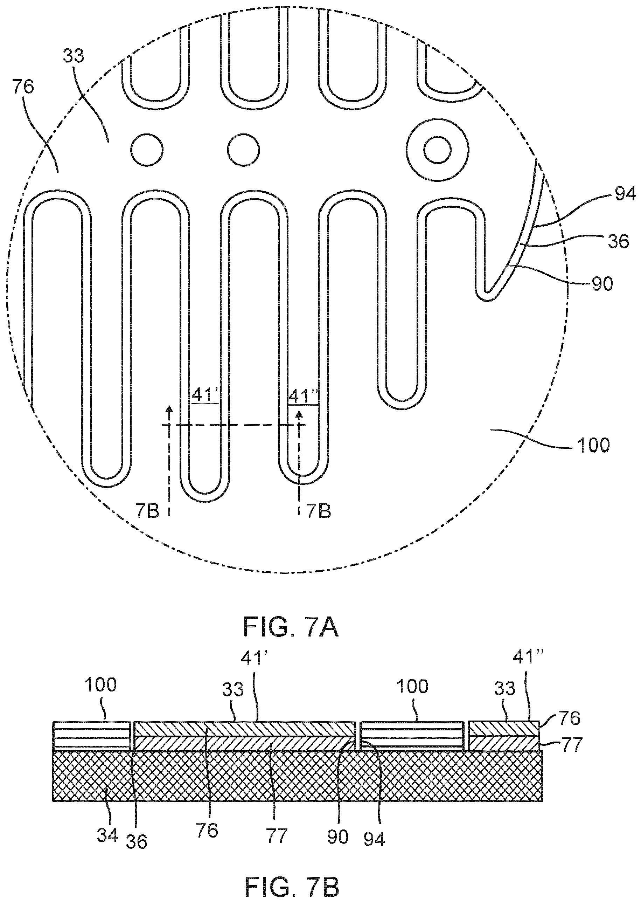

With reference to Figure 6, a coating 100 comprised of a dielectric material, e.g., parylene, is provided at least on the outer surface of membrane 26. The coating should be a dielectric material to help prevent electrical current from passing directly from one contact electrode 33 to another contact electrode 33. Coating 100 may additionally be provided on at least a portion of each of the substrates 34 of flexible circuits 84. Coating 100 may additionally be provided on a peripheral portion of contact electrode 33. As shown in Figure 6 and its alternate detail views D depicted in Figures 6A and 7A, coating 100 is provided such that it extends onto substrate 34, but in neither instance does it cover any portion of contact electrodes 33.

In Figure 6A, corresponding to detail view D in Figure 6, coating edge 94 is spaced from contact electrode 33, including its protrusions, such as first protrusion 41' and second protrusion 41". Accordingly, as seen in Figure 6B, which is a cross-section view taken through section line 6B-6B in Figure 6A, no portion of coating 100 is visible between adjacent protrusions, e.g., first protrusion 41' and second protrusion 41", thus leaving a gap 93 between these protrusions.

Alternatively, coating 100 may nearly abut, abut, or even slightly cover the outer boundary 90 of contact electrode 33, such that coating 100 is also disposed atop substrate 34 between adjacent protrusions of contact electrode 33, e.g., first protrusion 41' and second protrusion 41". As shown in Figure 7A, which corresponds alternatively to the detail D of Figure 6, and the cross-section view as depicted Figure 7B taken through section line 7B-7B in Figure 7A, coating edge 94 nearly abuts boundary 90 of contact electrode 33. Thus, the top surface of contact electrode 33 and coating 100 together make a fairly even or smooth surface, including between the protrusions of contact electrode 33, e.g., first protrusion 41' and second protrusion 41". Where coating 100 covers a peripheral portion of contact electrode 33, at least 98% of the surface area of contact electrode 33 should remain exposed, i.e., is not covered by coating 100, to ensure that coating 100 does not inhibit contact being made between contact electrode 33 and tissue, or otherwise increase the impedance of any electrical connection therebetween.

Coating 100 may have a thickness of between about one micron and about five microns, e.g., about three microns. The thickness of coating 100 may vary over the surface of balloon 80 depending on whether coating 100 is disposed only on membrane 26, only on membrane 26 and substrate 34 up to boundary 90 of contact electrode 33, or on all three of membrane 26, substrate 34, and a peripheral portion of contact electrode 33. Further, the thickness of coating 100 may depend on whether coating 100 should impart increased smoothness to the outer surface of balloon 80. Thus, coating 100 may be somewhat thicker over membrane 26 than substrate 34. Coating 100 may thus be provided such that its outer surface is disposed at or nearly at the same level as the outer surface of contact layer 76 of contact electrode 33. In other words, a difference between a first distance from the outer surface of membrane 26 to the outer surface of coating 100 and a second distance from the outer surface of membrane 26 to the outer surface of contact electrode is less than about 5 micrometers, e.g., less than about 1 micrometer or less than about 0.5 micrometers.

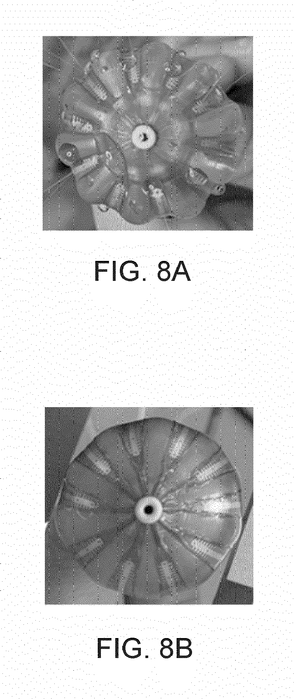

Balloon 80 should have an ellipsoidal, e.g., spherical, form when contact electrodes 33 are pressed against tissue during a procedure. Coating 100 assists balloon in maintaining its form when expanded because coating 100 increases the resilience of balloon 80. Applicant has demonstrated this, as depicted in Figures 8A and 8B, which comprise photographs of the bottom of two different specimens of a balloon 80 that is designed to have a spherical form with a diameter of 30 millimeters after being expanded by telescopic movement of distal shaft portion 82D proximally in proximal shaft portion 82P, described above, and then subject to an internal pressure of up to about one pound per square inch generated by filling balloon 80 with irrigation fluid via fluid connection 73. In Figure 8A, balloon 80 lacks coating 100 and has been expanded under a pressure of about 1.5 pounds per square inch. So expanded, balloon 80 has a scalloped, non-spherical shape. That is, membrane 26 bulges between contact electrodes 33 to create valleys in which contact electrodes 33 sit. The maximum diameter of this scalloped balloon, i.e., the distance from one bulge to a bulge on the opposite side of the balloon (180 degrees around the balloon), was measured to be about 31 millimeters, whereas the minimum diameter, i.e., the distance from one contact electrode 33 to a contact electrode 33 on the opposite side of the balloon, remained about 30 millimeters. Thus, a diameter variation of about 1 millimeter is observed in this expanded specimen of balloon 80 at an expansion pressure of about 1.5 pounds per square inch. This specimen of balloon 80 bursts at pressures greater than about two pounds per square inch.

In Figure 8B, balloon 80 includes coating 100 and has been expanded under a greater pressure of about 5.5 pounds per square inch, which is about four pounds per square inch more than the expansion pressure of the balloon in Figure 8A. Bursting has been avoided. Even under this drastic increase to the internal pressure inside balloon 80, the balloon has maintained a spherical shape with a maximum diameter of only 30.5 millimeters. Thus, a diameter variation of about 0.5 millimeters is observed in this expanded specimen of balloon 80, which is an increase of 1.7% over the intended diameter of 30 millimeters.

Accordingly, coating 100 increases the resilience of balloon 80. Coating 100 may prevent the diameter of membrane 26 from increasing by greater than about 0.5% when membrane 26 is subject to an internal pressure between about 1 pound per square inch and about 2.5 pounds per square inch. That is, under these conditions, the diameter of the membrane increases by less than about 0.5%. Coating 100 may prevent the diameter of membrane 26 from increasing by greater than about 1.25% when membrane 26 is subject to an internal pressure between about 2.5 pounds per square inch and about 4 pounds per square inch. That is, under these conditions, the diameter of the membrane increases by less than about 1.25%. Coating 100 may prevent the diameter of membrane 26 from increasing by greater than about 2% when membrane 26 is subject to an internal pressure between about 4 pounds per square inch and about 5.5 pounds per square inch. That is, under these conditions, the diameter of the membrane increases by less than about 2%. It should be understood that the aforementioned internal pressures generated in balloon 80 are less than a corresponding pressure at fluid connection 73 due to a pressure drop that occurs as the irrigation fluid passes though irrigation tubing that connects fluid connection 73 to balloon 80. Furthermore, the pressure generated in balloon 80 as a function of the pressure at fluid connection 73 depends on the number of irrigation pores 35 in balloon 80 as well as their size.

The dielectric properties of coating 100 also help prevent electrical current from passing directly from one electrode to another, which is particularly useful when the electrodes are used in a bipolar fashion, e.g., to perform ablative procedures via electroporation from electric fields created by pulsed high-voltage direct electrical current. Ablation of tissues during a surgical procedure, e.g., a pulmonary-vein isolation procedure, is commonly performed by providing radio frequency energy to cardiac tissues from electrodes disposed on a catheter, such as diagnostic/therapeutic catheter 24. The radio frequency energy heats the tissue to cause lesions therein. Care must be taken to ensure that the lesions are formed in the correct locations in diseased tissue and to avoid injuring non-diseased tissues. It has been determined that ablation of cardiac tissue, such as myocardial tissue, via irreversible electroporation provides certain advantages relative to heating from radio frequency energy. Notably, myocardial tissue is more likely to be damaged from electric fields resulting from pulsed high-voltage direct electrical current than other types of tissue, even including other types of cardiac tissues. Moreover, irreversible electroporation of myocardial tissue may be accomplished without heating the myocardial tissue, which is useful for minimizing any likelihood of injuring non-diseased tissue.

Applicant has determined that coating 100 improves the ability of balloon 80 to ablate tissue via electroporation from electric fields created by pulsed high-voltage direct electrical current, even when balloon 80 is not over-pressurized to create bulges and valleys. Applicant theorizes that this may be because the dielectric coating inhibits the direct current from flowing directly between two contact electrodes 33. Furthermore, the increased smoothness to balloon 80 imparted by coating 100 results in better conformance of balloon 80 to the pulmonary vein ostium and thus reduces the presence of blood or irrigation fluid (i.e., saline) at the interface between electrodes 30 and tissue. Specifically, when balloon 80 is positioned against tissue, a space may be defined between tissue and membrane 26 that is bound on opposite sides by two neighboring electrodes 30 if coating is not present. Blood and saline might collect in this space. Accordingly, by applying the dielectric coating on outer surface 26o of membrane 26, and perhaps also onto substrate 34, there should be less space for blood or saline to collect between the electrodes.

By inhibiting the direct current from flowing directly from one contact electrode 33 to another, e.g., through blood or saline, inside the balloon or out, the direct current and its corresponding electric field are shunted into the myocardial tissue. Such assists in forming lesions in myocardial tissue with sufficient size and uniformity to provide a therapeutic effect (e.g., isolating the pulmonary vein), while also reducing or avoiding certain risks associated with heating caused by RF ablation (e.g., burning non-target tissues).

An exemplary material for coating 100 has a relative permittivity of between about 2 and about 4, e.g., about 3, and a high resistance to stretching. For example, the dielectric coating may comprise any variation of parylene, such as parylene C, N, F.

Applicant has determined that lesions that are uniformly distributed throughout the myocardial tissue of the pulmonary vein ostium have the highest likelihood of isolating the pulmonary vein while also minimizing risk of injury to other target tissues. Such lesions may be enabled by using a version of balloon 80 that has been modified with coating 100. To create these lesions, direct current between a pair of electrodes 33 should be provided in short pulses at a high voltage. For example, between about one and about 300 direct-current pulses per second, e.g., 150 pulses, may be provided every about 0.1 seconds to about 10 seconds, e.g., about 5 seconds, and each pulse may have a voltage of about 1500 volts and about 3000 volts, e.g., 1800 volts. These direct-current pulses may be provided continuously for between about 1 second and about 100 seconds, e.g., about fifty seconds. The direct-current pulses may also be provided in sets. That is, after providing a first set of pulses having the characteristics just described, a second set of pulses having the same or different characteristics as the first set may be provided after not providing any pulses during a rest period that lasts for between about 0.1 seconds and about 10 seconds. For example, two such sets to twenty such sets, e.g., ten such sets, may be provided. Furthermore, the current in each pulse may be positive, negative, or alternate between positive and negative.

Any of the examples or embodiments described herein may include various other features in addition to or in lieu of those described above. The teachings, expressions, embodiments, examples, etc., described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A catheter balloon, comprising:
an ellipsoidal membrane including a proximal end and a distal end, and an outer surface;
a plurality of flexible circuit strips disposed about the membrane, the flexible circuit strips each comprising:
a substrate including an outer surface and an inner surface, the inner surface disposed atop the outer surface of the membrane, and
a contact electrode disposed on the outer surface of the substrate so that the contact electrode is exposable to an environment; and
a coating disposed atop the outer surface of the membrane, but not atop any of the contact electrodes.

2. The catheter balloon of claim 1, in which the plurality of flexible circuit strips each extend from the proximal end of the membrane toward the distal end of the membrane.

3. The catheter balloon of claim 2, in which the coating is disposed atop a portion of each of the substrates.

4. The catheter balloon of claim 3, in which each of the contact electrodes comprises a fishbone configuration having a plurality of protrusions extending away from a main body.

5. The catheter balloon of claim 4, in which a portion of the coating is disposed between a first protrusion and a second protrusion of the plurality of protrusions.

6. The catheter balloon of claim 3, in which the coating comprises a dielectric material.

7. The catheter balloon of claim 6, in which the dielectric material comprises parylene.

8. The catheter balloon of claim 6, in which a thickness of the coating is between about one micron and about five microns.

9. The catheter balloon of claim 8, in which the thickness of the coating is about three microns.

10. The catheter balloon of claim 6, in which a difference between a first distance from the outer surface of the membrane to an outer surface of the coating and a second distance from the outer surface of the membrane to the outer surface of the contact electrode is less than about 5 micrometers.

11. The catheter balloon of claim 10, in which the difference is less than about 1 micrometer.

12. The catheter balloon of claim 11, in which the difference is less than about 0.5 micrometers.

13. The catheter balloon of claim 1, in which the membrane has a first diameter when subject to a first internal pressure of less than about one pound per square inch, and in which the membrane has a second diameter when subject to a second internal pressure of greater than one pound per square inch.

14. The catheter balloon of claim 13, in which the second diameter is less than 0.5% greater than the first diameter when the second internal pressure is between about 1 pound per square inch and about 2.5 pounds per square inch.

15. The catheter balloon of claim 13, in which the second diameter is less than 1.25% greater than the first diameter when the second internal pressure is between about 2.5 pounds per square inch and about 4 pounds per square inch.

16. The catheter balloon of claim 13, in which the second diameter is less than 2 % greater than the first diameter when the second internal pressure is between about 4 pounds per square inch and about 5.5 pounds per square inch.

17. The catheter balloon of claim 14, in which the diameter equals about 30 millimeters.

18. The catheter balloon of claim 17, in which the plurality of flexible circuit strips comprises ten flexible circuit strips.
